# FASCICULE DE BREVET EUROPEEN

(11) **EP 1 830 632 B1**
(45) Date de publication et mention de la délivrance du brevet: **21.01.2009**
(21) Numéro de dépôt: 05848181.3
(22) Date de dépôt: 19.12.2005
(51) Int. Cl.: A01K 67/027, C12Q 1/68

(54) **PROCEDE D'OBTENTION D'UN HYBRIDE INTERSPECIFIQUE TRIPLOIDE DE BAR**
VERFAHREN ZUR HERSTELLUNG EINER TRIPLOIDEN ARTKREUZUNG DES SEEBARSCHES
METHOD FOR OBTAINING A TRIPLOID INTERSPECIFIC HYBRID SEA BASS

(30) Priorité: 21.12.2004 FR 0453129
(43) Date de publication de la demande: 12.09.2007
(73) Titulaire: INSTITUT FRANCAIS DE RECHERCHE POUR L'EXPLOITATION DE LA MER (IFREMER), 92130 Issy-les-Moulineaux Cedex (FR)
(72) Inventeur: KY, Chin Long, F-34980 Combaillaux (FR); Falguiere, Jean-Claude, F-97212 St Joseph La Martinique (FR)
(74) Mandataire: Catherine, Alain
(86) Numéro de dépôt international: PCT/FR2005/051109
(87) Numéro de publication internationale: WO 2006/067356

(56) Documents cités:
- WO-A-20/04080165
- PERUZZI STEFANO ET AL: "Production of meiotic gynogenetic and triploid sea bass, Dicentrarchus labrax L. 1. Performances, maturation and carcass quality." AQUACULTURE, vol. 230, no. 1-4, 16 février 2004 (2004-02-16), pages 41-64, XP002346275 ISSN: 0044-8486
- GARCIA DE LEON F J ET AL: "Development and use of microsatellite markers in sea bass, Dicentrarchus labrax (Linnaeus, 1758) (Perciformes: Serranidae)" MOLECULAR MARINE BIOLOGY AND BIOTECHNOLOGY, vol. 4, no. 1, 1995, pages 62-68, XP008052745 ISSN: 1053-6426 cité dans la demande
- GORSHKOV S ET AL: "Chromosome set manipulations and hybridization experiments in gilthead seabream (Sparus aurata). II. Assessment of diploid and triploid hybrids between gilthead seabream and red seabream (Pagrus major)." JOURNAL OF APPLIED ICHTHYOLOGY, vol. 18, no. 2, avril 2002 (2002-04), pages 106-112, XP002346276 ISSN: 0175-8659
- HALLERMAN ERIC M: "Toward coordination and funding of long-term genetic improvement programs for striped and hybrid bass Morone sp" JOURNAL OF THE WORLD AQUACULTURE SOCIETY, vol. 25, no. 3, 1994, pages 360-365, XP008052912 ISSN: 0893-8849

### SEQUENCE LISTING

<110> Institut français de Recherche pour l'Exploitation de la Mer (IFREMER)
<120> Procédé de réalisation d'un hybride interspécifique triploïde de bar
<130> R510FR
<160> 3
<170> PatentIn version 3.1
<210> 1
   <211> 46
   <212> DNA
   <213> Dicentrarchus labrax
<400> 1
   acacacacac acacacacac acacacacac acacacacac acacac 46
<210> 2
   <211> 20
   <212> DNA
   <213> Dicentrarchus labrax
<400> 2
   aaacagtctt tcaagtggtc 20
<210> 3
   <211> 20
   <212> DNA
   <213> Dicentrarchus labrax
<400> 3
   atggacaact gctgtcatag 20

## Description

La présente invention concerne le domaine de l'aquaculture, et en particulier, l'obtention d'un hybride interspécifique triploïde de bar.

L'augmentation de la population mondiale entraîne une demande sans cesse croissante de protéines de haute qualité, en particulier d'origine aquatique. On observe donc aujourd'hui, une augmentation de l'aquaculture pour répondre à cette demande, qui ne peut être satisfaite uniquement par la pêche industrielle classique. En effet, les capacités de production de poisson par la pêche industrielle classique arrivent à saturation ou sont en déclin, du fait de la surpêche, de la destruction de l'habitat des poissons, ou de la pollution.

L'aquaculture méditerranéenne a donc connu un grand succès ces dernières années. L'aquaculture du bar *(Dicentrarchus Labrax)* s'est beaucoup développée, permettant de faire diminuer substantiellement le prix de ce type de poisson. On observe donc aujourd'hui une demande croissante des consommateurs pour le bar, à l'image de ce qui a pu être constaté pour le saumon.

A titre d'exemple, en Europe, la production de bar *(Dicentrarchus labrax)* atteint environ 80000 tonnes par an et se hisse au second rang avec la daurade, après les salmonidés.

Le bar atteint sa taille commerciale de l'ordre de 400 grammes, au bout de 18 mois d'élevage dans des conditions optimales ; on le nomme alors « bar portion ». Ce délai est assez long par comparaison à d'autres espèces. De plus, le coût de production s'accroît avec la durée de l'élevage, ce qui induit une demande pressante d'amélioration des performances, principalement la croissance, pour augmenter la compétitivité des entreprises. Par ailleurs, les producteurs européens de bar recherchent des moyens de stériliser leur cheptel pour :
- protéger le progrès génétique induit par leur programme de sélection, et
- limiter le risque de pollution génétique des stocks sauvages de bar par des individus échappés des structures d'élevage.

Le document par Peruzzi S et al (2004) "Production of meiotic gynogenetic and triploid sea bass, Dicentrarchus labrax L. 1. Performances, maturation and carcass quality" AQUACULTURE, vol. 230, no. 1-4, 16 février 2004 (2004-02-16), pages 41-64, ISSN: 0044-8486, décrit, pour les poissons D.labrax triploïdes obtenus par choc hyperbare, les caractéristiques phénotypiques relatives aux performances, à la maturation et à la qualité de la carcasse. Sont décrites notamment une proportion élevée de mâles, une maturation sexuelle déficiente, et la stérilité des triploïdes obtenus. La croissance à 34 mois est inférieure pour les triploïdes par rapport aux diploïdes.

Il existe ainsi un besoin pour la production de nouveaux bars stériles, dit « à haute performance de croissance » dont la taille commerciale minimale est atteinte plus rapidement que celle des animaux élevés habituellement.

Les inventeurs ont mis au point un procédé d'obtention d'un hybride interspécifique triploïdie de bar comprenant les deux étapes suivantes :
(i) croiser un bar *D. labrax* avec un bar *D*. *punctatus,* et
(ii) réaliser un traitement d'induction de la triploïdie sur la descendance obtenue.

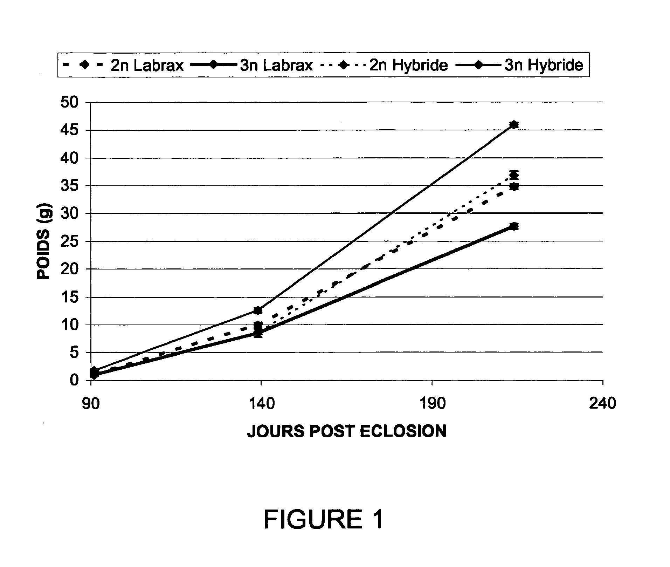

Le procédé mis au point par les inventeurs permet d'obtenir des animaux dont la prise de poids est plus rapide que celle des animaux élevés habituellement. La croissance rapide des bars hybrides triploïdes *D. labraxlD. punctatus* par rapport à celle des bars *D. labrax* a été mise en évidence dans l'exemple 1, illustré par la figure 1.

Le procédé de l'invention permet d'obtenir des animaux stériles, ce qui permet d'obéir aux recommandations des institutions internationales préconisant l'élevage de poissons stériles afin d'éviter les reproductions non contrôlées entre les souches domestiquées d'élevage et les stocks sauvages du milieu naturel et ainsi préserver la biodiversité. La production d'animaux stériles permet enfin d'améliorer et de stabiliser la qualité des animaux produits grâce à l'absence de développement des gonades. La maturation sexuelle est en effet responsable de modifications de la physiologie du poisson induisant une dégradation de la qualité du produit.

Le croisement interspécifique permet, en outre, d'éviter les problèmes constatés habituellement avec les croisements intraspécifiques, comme la perte de diversité génétique entraînant à terme des pertes conséquentes de production.

L'hybridation interspécifique permet également, au delà de l'augmentation de la vitesse de croissance, d'améliorer la résistance aux maladies, ou de pouvoir manipuler les ratios entre les sexes des animaux.

Enfin, le procédé de l'invention, évite le recours à des techniques de génie génétique, ce qui constitue un avantage technique supplémentaire vis-à-vis de l'innocuité de l'aliment envers le consommateur final, et la possibilité de commercialiser les bars triploïdes de l'invention sans requérir d'autorisation administrative préalable.

Le procédé d'obtention d'un hybride interspécifique triploïdie de bar défini ci-dessus n'est en aucun cas un procédé essentiellement biologique. En effet, l'une des étapes essentielles du procédé consiste à réaliser un traitement d'induction de la triploïdie. Cette étape, impossible sans intervention humaine, a un impact décisif sur l'obtention d'une population d'individus triploïdes permettant une exploitation industrielle.

De préférence, l'étape (ii) de traitement d'induction de la triploïdie consiste à soumettre les oeufs fécondés à un choc hyperbare.

De préférence, le choc hyperbare est réalisé à une pression de 400 à 800 bars pendant 1 à 4 minutes.

Alternativement, l'étape (ii) de traitement d'induction de la triploïdie consiste à soumettre les oeufs fécondés à un choc thermique, de préférence à une température de -5°C à +5°C pendant 10 à 30 minutes.

De manière générale, le traitement d'induction de la triploïdie est réalisé 2 à 10 minutes après fécondation des oeufs.

Dans la pratique, il n'y a aucune nécessité de rechercher, parmi les bars obtenus selon le procédé de l'invention, lesquels sont des bars hybrides triploïdes, du fait que la population de bars obtenus selon le procédé est constitué de bars hybrides triploïdes à approximativement 100 %.

Toutefois, dans un mode de réalisation particulier, le procédé de l'invention comprend l'étape supplémentaire suivante :
(iii) sélectionner le ou les individus hybrides triploïdes au sein de la descendance obtenue.

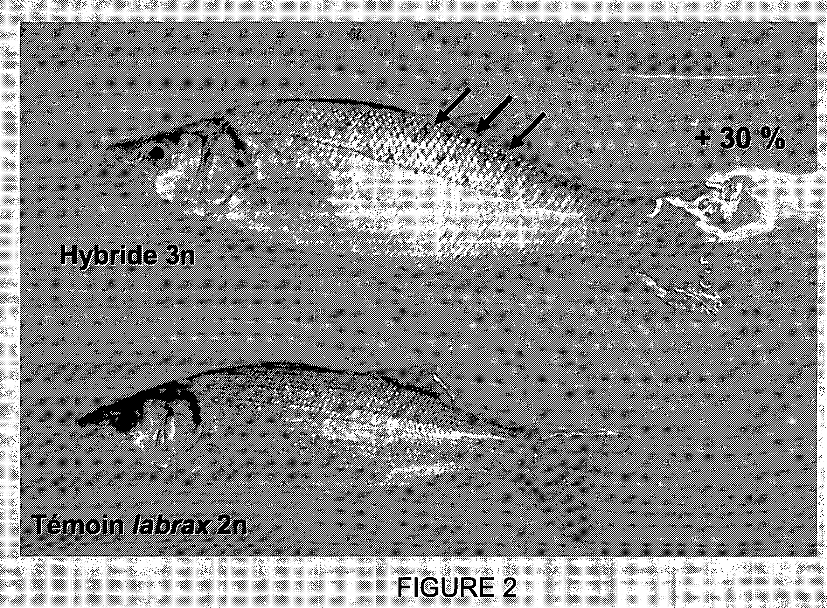

La sélection des bars hybrides peut s'effectuer de manière très simple, par contrôle visuel. En effet, les bars hybrides possèdent des différences phénotypiques par rapport aux bars sauvages. Comme cela est illustré figure 2, les bars hybrides et les bars *D. labrax* présentent localement des différences, par exemple les bars hybrides possèdent une robe ponctuée que ne possèdent pas les bars *D. labrax.*

La sélection des individus hybrides triploïdes peut également être réalisée génétiquement, selon deux étapes distinctes pouvant être réalisées dans un ordre quelconque.

La première étape consiste à sélectionner les individus triploïdes. A titre d'exemple la détermination de la ploïdie peut être réalisée par analyse des cellules d'un bar à tester, en cytométrie de flux comme cela est décrit dans l'exemple 4 ou en réalisant un caryotype comme cela est illustré dans l'exemple 5.

La seconde étape consiste à sélectionner les individus hybrides parmi les individus triploïdes. A titre d'exemple la détermination du caractère hybride des bars peut être réalisée en recherchant la présence simultanée au sein d'un bar à tester :
- d'au moins un marqueur génétique (a) présent chez *D*. *labrax* et absent chez *D. punctatus,* et
- d'au moins un marqueur génétique (b) présent chez *D*. *punctatus* et absent chez *D. labrax.*
La détection de tels marqueurs sera décrite plus en détails dans la suite de la description et dans les exemples 6 et 7.

Inversement, les bars triploïdes peuvent également être sélectionnés parmi les bars hybrides. Le choix de la séquence de ces deux étapes dépendra en particulier du coût ou de la difficulté des méthodes choisies pour déterminer le caractère hybride et la ploïdie.

De préférence, le procédé selon l'invention comprend les deux étapes suivantes :
(i) croiser un bar *D. labrax* femelle avec un bar *D. punctatus* mâle, et
(ii) réaliser un traitement d'induction de la triploïdie sur la descendance obtenue.

Alternativement, le procédé selon l'invention comprend les deux étapes suivantes :
(i) croiser un bar *D. labrax* mâle avec un bar *D*. *punctatus* femelle, et
(ii) réaliser un traitement d'induction de la triploïdie sur la descendance obtenue.

L'invention concerne également des hybrides triploïdes de bar, comprenant :
- au moins un marqueur génétique (a) présent chez *D*. *labrax* et absent chez *D. punctatus,* et
- au moins un marqueur génétique (b) présent chez *D*. *punctatus* et absent chez *D. labrax.*

Par marqueur génétique, on entend toute séquence d'ADN ou tout nucléotide spécifiquement repérable chez un individu, mais également toute caractéristique morphologique ou biochimique permettant de différencier un bar *D. labrax* d'un bar *D. punctatus,* par exemple, une différence de robe.

En particulier, les marqueurs génétiques utilisés peuvent être des marqueurs moléculaire de l'ADN.

A titre d'exemple de marqueur moléculaire, on peut citer les techniques fournissant des marqueurs codominants révélés individuellement comme la RFLP (Restriction Fragment Length Polymorphism), les microsatellites ou SSR (single séquence repeat) et les techniques fournissant en masse des marqueurs dominants, comme la RAPD (Random Amplified Polymorphic DNA), l'AFLP (Amplified Fragment Length Polymorphism). La validation du caractère hybride des bars peut s'effectuer selon l'une quelconque de ces techniques.

Ainsi, de préférence, les marqueurs génétiques (a) et (b) sont des marqueurs moléculaires choisis parmi les séquences d'ADN microsatellites, les fragments d'ADN issus de RFLP, d'AFLP, ou de RAPD.

La détection génétique du marqueur peut s'effectuer, par exemple, par hybridation avec une sonde complémentaire, ou par son expression phénotypique. La détection du marqueur génétique permet de discriminer un bar hybride triploïde, issu du croisement de *D. labrax* avec *D*. *punctatus,* au sein d'une population de poissons.

La détection du marqueur génétique peut s'effectuer en révélant en masse des marqueurs dominants, par exemple selon le principe de l'AFLP (Ky et al., 2000).

Brièvement, cette méthode consiste à effectuer une digestion de l'ADN génomique d'un bar par deux enzymes de restriction, dont la fréquence de coupure est différente. On choisit par exemple *M*seI et *Eco*RI. Les fragments résultant de cette coupure sont ensuite soumis à une ligation avec des séquences nucléotidiques adaptateurs s'hybridant spécifiquement aux extrémités des fragments. Une amplification en chaîne par réaction (PCR) est ensuite réalisée en utilisant des amorces dont les séquences sont complémentaires des séquences nucléotidiques adaptateurs, et comprennent une base additionnelle en 3'. Une seconde PCR est réalisée sur les fragments issus de la première amplification selon le même principe que précédemment, avec des amorces comprenant deux bases additionnelles. Une électrophorèse sur gel d'acrylamide est ensuite réalisée de manière classique pour permettre de distinguer les fragments amplifiés en fonction de leurs tailles respectives.

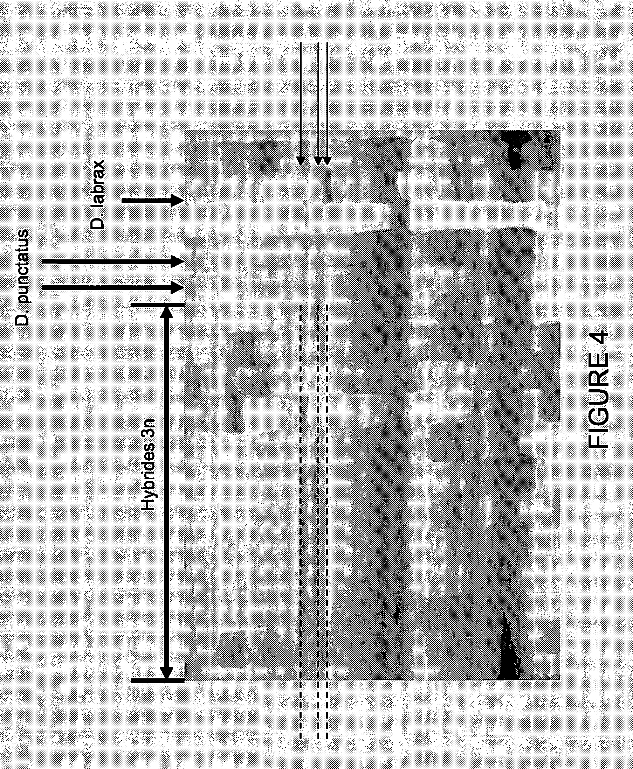

En fonction de la position et de la présence de chaque fragment sur le gel, il est possible de savoir si le bar testé est un hybride issu du croisement D *labrax D. punctatus.* En effet, certains fragments observés sont des marqueurs spécifiques de l'espèce *D. labrax,* non présents chez *D*. *punctatus,* marqueurs définis ci-dessus comme marqueurs de type (a). Inversement certains fragments observés sont des marqueurs spécifiques de l'espèce *D. punctatus ,* non présents chez *D. labrax,* il s'agit de marqueurs définis ci-dessus comme marqueurs de type (b). Si les marqueurs de type (a) et de type (b) sont présents chez un bar, on peut conclure qu'il s'agit d'un hybride issu du croisement D *labrax D. punctatus.* Le résultat d'une détection de type AFLP est illustré dans l'exemple 6, et sur la figure 4.

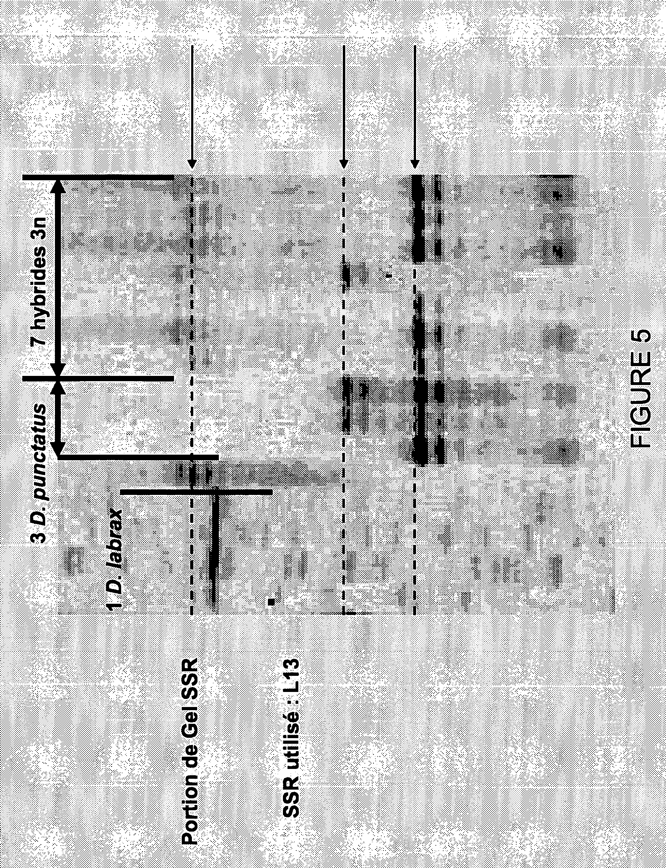

Une autre méthode de détection de marqueurs génétiques peut être envisagée pour discriminer un bar hybride, issu du croisement de *D. labrax* avec *D. punctatus,* au sein d'une population de poissons. Il est possible révéler individuellement des marqueurs codominants, par exemple, en ayant recours aux marqueurs de type microsatellites selon le principe de la SSR. (exemple 7, figure 5).

Cette méthode consiste à identifier au sein du génome d'un bar, la présence de marqueurs de type (a) ou (b) sous la forme de courtes séquences d'ADN répétées en tandem dans le génome des bars.

En particulier, les marqueurs génétiques (a) peuvent être des séquences d'ADN microsatellites choisies parmi les séquences suivantes : Labrax-3, Labrax-6, Labrax-8, Labrax-9, Labrax-13, Labrax-17, ou Labrax-29.

De préférence, le marqueur génétique (a) est le marqueur Labrax-13, de séquence SEQ ID N°1. (Garcia de Leon, 1995).

Le marqueur Labrax-13 consiste en un fragment d'ADN répété qui est bordé, à ses extrémités, de séquences hybridant respectivement avec les amorces de séquences SEQ ID N°2 et SEQ ID N°3. Chez *D.labrax* et *D. punctatus,* respectivement, l'utilisation du couple d'amorces SEQ ID N°2 et SEQ ID N°3 permet d'amplifier des fragments d'ADN répété dont la longueur est caractéristique de l'une ou l'autre espèce. A titre illustratif, l'utilisation de ce couple d'amorces avec l'ADN génomique de *D. labrax* permet d'amplifier un fragment d'ADN de séquence SEQ ID N°1.

La première étape de cette méthode de détection consiste à effectuer une amplification en chaîne par réaction (PCR) des microsatellites décrits ci-dessus en utilisant des amorces spécifiques. Dans le cas de l'amplification de Labrax-13, les amorces utilisées sont des séquences SEQ ID n°2 et SEQ ID N°3.

Les séquences issues des amplifications en chaîne par réaction (PCR) sont ensuite déposées sur un gel d'acrylamide en vue d'une électrophorèse. Les différentes séquences sont ensuite séparées en fonction de leurs tailles respectives.

La (les) taille(s) des fragments d'ADN amplifiés permet de déterminer comme pour la méthode décrite précédemment, si le bar testé est un hybride issu du croisement D *labrax D. punctatus.* Le résultat d'une détection d'ADN microsatellite est illustré dans l'exemple 7, et sur la figure 5.

Les méthodes de détection de marqueurs, illustrées ci-dessus sont très différentes l'une de l'autre. D'une manière générale, toutes les méthodes de détection de marqueurs génétiques connues de l'homme du métier peuvent être utilisées pour discriminer un bar hybride répondant à la définition de l'invention au sein d'une population de bars.

La discrimination des bars hybrides peut également s'effectuer de manière très simple, par un simple contrôle visuel. En effet, les hybrides possèdent des différences importantes par rapport aux bars sauvages. Comme cela est illustré figure 2, les bars hybrides triploïdes possèdent une robe ponctuée que ne possèdent pas les bars *D. labrax.*

### Description des figures

**Figure 1** **:** Croissance pondérale (en grammes) en fonction du temps (nombre de jours post-éclosion) de 4 lots de bars (moyenne de 32 individus par lot). L'évolution du poids moyen de bars *D. labrax* diploïdes (2nlabrax), et triploïdes (3nLabrax), et de bars hybrides diploïdes (2nHybride) et triploïdes (3nhybride) a été mesurée au cours du temps.
**Figure 2** **:** Photo illustrant l'hybride *D. labraxlD. punctatus* triploïde (en haut) et le témoin *D. labrax* diploïde (en bas).
**Figure 3** **:** Photos illustrant les caryotypes réalisés au niveau des espèces parentales, *D. labrax* (2n=48) (A), *D. punctatus* (2n=48) (B), et l'hybride triploïdie *D. labraxlD. punctatus* (3n=72) (C).
**Figure 4** **:** Portion de gel AFLP utilisant le couple d'amorces : E*-ACG/M-CAC.
   Piste 16 : *D.labrax*
   Pistes 13 et *14 : D. punctatus*
   Pistes 1 à 12 : hybrides *D. labraxlD. punctatus.*
**Figure 5** **:** Portion de gel SSRP utilisant le marqueur Labrax-13 de séquence SEQ ID N°1.
   Piste 5 : *D. labrax*
   Pistes 6 à 8 : *D. punctatus*
   Pistes 9 à 15 : hybrides *D. labraxlD. punctatus.*

### Exemple 1 : Réalisation d'un croisement D. labrax D. punctatus

Les conditions d'élevage des géniteurs, l'induction de la maturation des gamètes par le contrôle de l'environnement et l'induction hormonale de la ponte ont été décrites par Carillo et al (1995). les conditions de la reproduction artificielle ont été précisées par Colombo et al (1995). Le croisement est réalisé selon les étapes suivantes :
- Sélection de femelles *D. labrax* présentant un stade de maturation ovocytaire adéquat et de mâles *D*. *labrax* et *D. punctatus* fluents.
- Injection de la femelle avec du LHRHa à la dose de 10 mg/kg de poids vif et isolement en structure de ponte individuelle thermorégulé.
- Récupération des gamètes femelles et mâles par stripping 70 h environ après l'injection de la femelle.
- Fécondation artificielle d'une partie des ovules labrax par du sperme labrax et d'une autre partie par du sperme punctatus
- Le mélange des oeufs, du sperme et de l'eau de mer se fait dans les proportions 1000 : 1 : 500.
- Une partie des oeufs fécondés n'est pas soumise au traitement d'induction de la triploïdie pour servir de contrôle diploïde.
- Une autre partie subit un traitement d'induction de la triploïdie par choc hyperbare ou thermique (Peruzzi and Chatain, 2000, Colombo et al, 1995).

Les larves écloses sont élevées selon les techniques classiques utilisées dans toutes les écloseries de bar à raison d'une densité initiale de 100 larves par litre d'eau. Elles sont nourries à partir d'un âge de 9 jours avec des artémias puis sont sevrées sur micro granulés entre 40 et 45 jours d'age. Pendant le prégrossissement, les poissons sont nourris avec du granulé commercial titrant 55 à 60% de protéines et 12 à 15% de lipides. Ils sont élevés à la densité initiale de 700 alevins par bac de 500 litres à 42 jours et cette densité est diminuée au cours de la croissance à 430 alevins par bac à 91 jours pour ne plus être modifiée jusqu'à la fin de l'expérience.

### Exemple 2 : Comparaison de la croissance d'un hybride triploïde de bar avec celle d'un bar D. labrax diploïde ou triploïde

On observe que les hybrides triploïdes de bar, issus d'un croisement *D. labrax* x *D. punctatus* présentent une croissance pondérale plus rapide que les trois autres catégories de bars :
- les bars *D. labrax* diploïdes,
- les bars *D. labrax* triploïdes, et
- les bars hybrides *D. labrax x D. punctatus* diploïdes.
Les résultats expérimentaux sont représentés sur la figure 1. Pour chaque point de la courbe de croissance, le poids correspond à la moyenne de la mesure de 32 individus prélevés de manière aléatoire.

### Exemple 3 : discrimination phénotypique des bars hybrides triploïdes.

Les bars hybrides triploïdes, issus du croisement *D. labrax D. punctatus,* sont reconnaissables visuellement du fait de leur morphologie. En particulier, comme cela est clairement exposé sur la figure 2, les bars hybrides triploïdes possèdent un excédent de chair situé sous la nageoire dorsale que ne possèdent pas les bars *D. labrax.*

### Exemple 4 : Détermination de la ploïdie par cytométrie de flux

La ploïdie d'environ 10 larves de bar, au stade 72 heures après fécondation a été déterminée. Chaque larve est placée individuellement dans un tube jetable, congelé en attente de l'analyse, et préparé en vue d'une analyse en cytométrie de flux à l'iodure de propidium (Tiersch et al., 1989).

La ploïdie est déterminée en utilisant un cytomètre de flux de type FACscan (Becton Dickinson). Des échantillons de larves de bar diploïdes sont utilisés pour servir de témoin.

Le contenu en ADN des cellules de chaque larve à tester est mesuré et comparé au contenu en ADN des cellules des échantillons témoins, ce qui permet de déterminer si les larves étudiées sont diploïdes ou triploïdes.

### Exemple 5 : Détermination de la ploïdie par caryotype

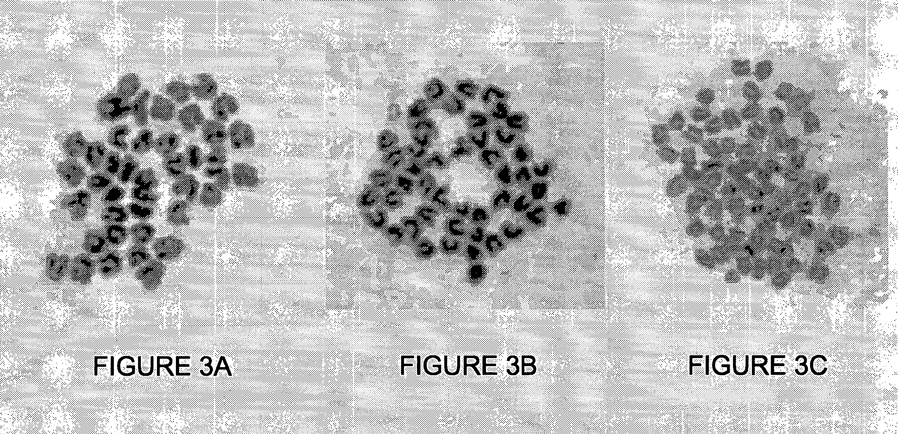

Un caryotype a été réalisé selon une méthode classique. Les caryotypes d'un bar hybride triploïdie (A), d'un bar *D. labrax* (B), et d'un bar *D. punctatus* (C) sont représentés Figure 3. Les bars hybrides triploïdes possèdent 3n=72 chromosomes et sont clairement distincts des bars D *labrax* et D *punctatus* possédant 2n=48 chromosomes.

### Exemple 6 : Validation du caractère hybride par AFLP

L'extraction d'ADN, les digestions enzymatiques et les amplifications par PCR de bars hybrides triploïdes, de bars *D. labrax* et de bars *D. punctatus* ont été effectuées selon la méthode décrite par Ky et al., (2000).

Comme on l'observe sur la figure 4, les individus hybrides, issus du croisement de *D. labrax* avec *D. punctatus* sont identifiables. Les hybrides sont en effet les seuls individus à posséder
- les marqueurs spécifiques de l'espèce *D. labrax,* non présents chez *D. punctatus,* (marqueurs de type (a)) et
- les marqueurs spécifiques de l'espèce *D. punctatus ,* non présents chez *D. labrax,* (marqueurs de type (b))
Il est donc aisé de déterminer si un bar répond à la définition de l'invention en effectuant le test de détection par AFLP

### Exemple 7 : Validation du caractère hybride par détection d'ADN microsatellites

L'extraction d'ADN, l'amplification par PCR de la séquence d'ADN microsatellite Labrax-13 de séquence SEQ ID N°1, et le génotypage de 7 hybrides *triploïdes, 1* bar *D*. *labrax,* et 3 bars *D. punctatus* ont été effectués selon la méthode décrite par Garcia de Leon et al. (1995). Les amorces utilisées sont des séquences SEQ ID N°2 et SEQ ID N°3.

Comme on l'observe sur la figure 5, les individus hybrides, issus du croisement de *D. labrax* avec *D. punctatus* sont clairement identifiables. Les hybrides sont en effet les seuls individus à posséder des séquences spécifiques de *D*. *labrax,* absentes chez *D. punctatus* et inversement.

Il est donc aisé de savoir si un bar répond à la définition de l'invention en effectuant le test de détection d'ADN microsatellite décrit ci-dessus.

### Références bibliographiques

Ky et al., (2000) Interspecific genetic linkage map, ségrégation distortion and genetic conversion in coffee (coffea sp.) TAG Theoritical and Applied Genetics, volume 101, number 4 pages 669-676.
Garcia de Leon et al., (1995) Development and use of microsatellite markers in sea bass, Dicentrarchus labrax (Linnaeus, 1758) (Perciformes : Serrandidae) Molecular Marine Biotechnology. 4(1), pp 62-68
Carillo, M., Zanuy, S., Prat, F., Cerda, J., Mananos, E. and Bromage, N., 1995. Sea bas (Dicentrarchus labrax). In : Bromage, N, Roberts, J. (Eds.), Broodstock Management an Eg and Larval Quality. Blackwell Science, Oxford, pp. 138-169.
Colombo, L., Barbaro, A., Libertini, A., Benedetti, P., Francescon, A. and Lombardo, I., 1995. Artificial fertilization and induction of triploidy and meiogynesis in the European sea bass, Dicentrarchus labrax. J; Appl. Ichthyol. 11 : pp. 118-125.
Peruzzi, S. and Chatain, B., 2000. Pressure and cold shock induction of meiotic gynogenesis and triploidy in the European sea bass Dicentrarchus labrax : relative efficiency of methods and parental variability. Aquaculture 189, pp. 23-37.
Tiersch, T.R., Chandler, R.W., Kallman, K.D. and Wachtel, S.S., 1989. Estimation of DNA nuclear content by flow cytometry in fishes of the genus Xyphophorus. Comparative biochemistry and physiology Part B : Biochemistry and molecular biology. 94 (3), pp 465-468.

## Revendications

1. Procédé d'obtention d'un hybride interspécifique triploïde de bar comprenant les deux étapes suivantes :
(i) croiser un bar *D. labrax* avec un bar *D*. *punctatus,* et
(ii) réaliser un traitement d'induction de la triploïdie sur la descendance obtenue.

2. Procédé selon la revendication 1, **caractérisé en ce que** l'étape (ii) de traitement d'induction de la triploïdie consiste à soumettre les oeufs fécondés à un choc hyperbare.

3. Procédé selon la revendication 2, **caractérisé en ce que** le choc hyperbare est réalisé à une pression de 400 à 800 bars pendant 1 à 4 minutes.

4. Procédé selon la revendication 1, **caractérisé en ce que** l'étape (ii) de traitement d'induction de la triploïdie consiste à soumettre les oeufs fécondés à un choc thermique.

5. Procédé selon la revendication 4, **caractérisé en ce que** le choc thermique est réalisé à une température de -5°C à +5°C pendant 10 à 30 minutes.

6. Procédé selon l'une quelconque des revendications 1 à 5, **caractérisé en ce que** l'étape (ii) de traitement d'induction de la triploïdie est réalisé 2 à 10 minutes après fécondation des oeufs.

7. Procédé selon l'une quelconque des revendications 1 à 5, **caractérisé en ce qu'**il comprend l'étape supplémentaire suivante :
(iii) sélectionner le ou les individus hybrides triploïdes au sein de la descendance obtenue.

8. Procédé selon l'une quelconque des revendications 1 à 5, comprenant les deux étapes suivantes :
(i) croiser un bar *D. labrax* femelle avec un bar *D*. *punctatus* mâle, et
(ii) réaliser un traitement d'induction de la triploïdie sur la descendance obtenue.

9. Procédé selon l'une quelconque des revendications 1 à 5, comprenant les deux étapes suivantes :
(i) croiser un bar *D. labrax* mâle avec un bar *D*. *punctatus* femelle, et
(ii) réaliser un traitement d'induction de la triploïdie sur la descendance obtenue.

10. Hybride triploïde de bar, **caractérisé en ce qu'**il comprend :
- au moins un marqueur génétique (a) présent chez *D*. *labrax* et absent chez *D. punctatus,* et
- au moins un marqueur génétique (b) présent chez *D*. *punctatus* et absent chez *D. labrax.*

11. Hybride triploïdie de bar selon la revendication 10, **caractérisé en ce que** les marqueurs génétiques (a) et (b) sont choisis parmi les séquences d'ADN microsatellites, les fragments d'ADN issus de RFLP, d'AFLP, ou de RAPD.

12. Hybride triploïde de bar selon la revendication 10 ou 11, **caractérisé en ce qu'**il comprend un marqueur génétique (a) Labrax-13, de séquence SEQ ID N°1.

## Claims

1. A method for obtaining a triploid interspecific hybrid sea bass comprising the following two steps:
(i) crossing a sea bass *D. labrax* with a sea bass *D. punctatus,* and
(ii) carrying out a treatment for inducing triploidy on the obtained progeny.

2. A method according to claim 1, **characterized in that** the step (ii) of treatment for inducing triploidy consists in subjecting the fertilized eggs to a hyperbaric shock.

3. A method according to claim 2, **characterized in that** the hyperbaric shock is performed at a pressure of from 400 to 800 bars for 1 to 4 minutes.

4. A method according to claim 1, **characterized in that** the step (ii) of treatment for inducing triploidy consists in subjecting the fertilized eggs to a thermal shock.

5. A method according to claim 4, **characterized in that** the thermal shock is performed at a temperature of from -5°C to +5°C for 10 to 30 minutes.

6. A method according to anyone of claims 1 to 5, **characterized in that** the step (ii) of treatment for inducing triploidy is performed at 2 to 10 minutes after egg fertilization.

7. A method according to anyone of claims 1 to 5, **characterized in that** it comprises the following additional step:
(iii) selecting the triploid hybrid individual(s) within the obtained progeny.

8. A method according to anyone of claims 1 to 5, comprising the following two steps:
(i) crossing a female sea bass *D. labrax* with a male sea bass *D. punctatus,* and
(ii) carrying out a treatment for inducing triploidy on the obtained progeny.

9. A method according to anyone of claims 1 to 5, comprising the following two steps:
(i) crossing a male sea bass *D. labrax* with a female sea bass *D. punctatus,* and
(ii) carrying out a treatment for inducing triploidy on the obtained progeny.

10. Triploid hybrid of sea bass, **characterized in that** it comprises:
- at least one genetic marker (a) present in *D. labrax* and absent in *D*. *punctatus,* and
- at least one genetic marker (b) present in *D. punctatus* and absent in *D. labrax.*

11. Triploid hybrid of sea bass according to claim 10, **characterized in that** the genetic markers (a) and (b) are selected from microsatellite DNA sequences, DNA fragments obtained from RFLP, AFLP, or RAPD.

12. Triploid hybrid of sea bass according to claim 10 or 11, **characterized in that** it comprises a genetic marker (a) Labrax-13, of the sequence SEQ ID N°1.

## Patentansprüche

1. Verfahren zur Gewinnung eines triploiden interspezifischen Hybriden des Seebarschs, das die beiden folgenden Schritte umfasst:
(i) Kreuzen eines Seebarschs *D. labrax* mit einem Seebarsch *D*. *punctatus;* und
(ii) Durchführen einer Behandlung zur Induktion der Triploidie bei den erhaltenen Nachkommen.

2. Verfahren gemäß Anspruch 1, **dadurch gekennzeichnet, dass** Schritt (ii) der Behandlung zur Induktion der Triploidie darin besteht, die befruchteten Eier einem Hochdruckschock zu unterziehen.

3. Verfahren gemäß Anspruch 2, **dadurch gekennzeichnet, dass** der Hochdruckschock bei einem Druck von 400 bis 800 bar während 1 bis 4 Minuten durchgeführt wird.

4. Verfahren gemäß Anspruch 1, **dadurch gekennzeichnet, dass** Schritt (ii) der Behandlung zur Induktion der Triploidie darin besteht, die befruchteten Eier einem thermischen Schock zu unterziehen.

5. Verfahren gemäß Anspruch 4, **dadurch gekennzeichnet, dass** der thermische Schock bei einer Temperatur von -5 °C bis +5 °C während 10 bis 30 Minuten durchgeführt wird.

6. Verfahren gemäß einem der Ansprüche 1 bis 5, **dadurch gekennzeichnet, dass** Schritt (ii) der Behandlung zur Induktion der Triploidie 2 bis 10 Minuten nach Befruchtung der Eier durchgeführt wird.

7. Verfahren gemäß einem der Ansprüche 1 bis 5, **dadurch gekennzeichnet, dass** es den folgenden zusätzlichen Schritt umfasst:
(iii) Selektieren des oder der triploiden hybriden Individuen innerhalb der erhaltenen Nachkommen.

8. Verfahren gemäß einem der Ansprüche 1 bis 5, das die beiden folgenden Schritte umfasst:
(i) Kreuzen eines weiblichen Seebarschs *D. labrax* mit einem männlichen Seebarsch *D. punctatus;* und
(ii) Durchführen einer Behandlung zur Induktion der Triploidie bei den erhaltenen Nachkommen.

9. Verfahren gemäß einem der Ansprüche 1 bis 5, das die beiden folgenden Schritte umfasst:
(i) Kreuzen eines männlichen Seebarschs *D. labrax* mit einem weiblichen Seebarsch *D. punctatus;* und
(ii) Durchführen einer Behandlung zur Induktion der Triploidie bei den erhaltenen Nachkommen.

10. Triploider Hybrid des Seebarschs, **dadurch gekennzeichnet, dass** er Folgendes umfasst:
- wenigstens einen genetischen Marker (a), der bei *D. labrax* vorhanden ist und bei *D. punctatus* fehlt; und
- wenigstens einen genetischen Marker (b), der bei *D. punctatus* vorhanden ist und bei *D. labrax* fehlt.

11. Triploider Hybrid des Seebarschs gemäß Anspruch 10, **dadurch gekennzeichnet, dass** die genetischen Marker (a) und (b) aus Mikrosatelliten-DNA-Sequenzen, aus RFLP, AFLP oder RAPD hervorgegangenen DNA-Fragmenten ausgewählt sind.

12. Triploider Hybrid des Seebarschs gemäß Anspruch 10 oder 11, **dadurch gekennzeichnet, dass** er einen genetischen Marker (a) Labrax-13 der Sequenz SEQ ID Nr. 1 umfasst.
